(19) Europäisches Patentamt
European Patent Office
Office européen des brevets

(11) EP 3 165 231 A1

(12) EUROPEAN PATENT APPLICATION
published in accordance with Art. 153(4) EPC

(43) Date of publication:
**10.05.2017 Bulletin 2017/19**

(21) Application number: **15785504.0**

(22) Date of filing: **29.04.2015**

(51) Int Cl.:
***A61K 36/539*** *(2006.01)* ***A61K 8/97*** *(2017.01)*
***A61P 17/00*** *(2006.01)*

(86) International application number:
**PCT/KR2015/004291**

(87) International publication number:
**WO 2015/167240 (05.11.2015 Gazette 2015/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **30.04.2014 KR 20140052274**

(71) Applicant: **Amorepacific Corporation**
**Seoul 140-777 (KR)**

(72) Inventors:
- **PARK, Pil Joon**
  **Yongin-si**
  **Gyeonggi-do 446-729 (KR)**
- **LEE, So Hee**
  **Yongin-si**
  **Gyeonggi-do 446-729 (KR)**
- **CHO, Eun Gyung**
  **Yongin-si**
  **Gyeonggi-do 446-729 (KR)**
- **LEE, Tae Ryong**
  **Yongin-si**
  **Gyeonggi-do 446-729 (KR)**

(74) Representative: **Agasse, Stéphane et al**
**Cabinet GERMAIN & MAUREAU**
**B.P. 6153**
**69466 Lyon Cedex 06 (FR)**

# (54) COMPOSITION CONTAINING SCUTELLARIA ALPINA EXTRACT

(57) The present invention relates to a composition containing a Scutellaria alpina extract as an active ingredient. The composition of the present invention exhibits effects of reducing the amount of endothelin 1 by containing a Scutellaria alpine extract, and reducing the overexpression of NK1R and EDN1 genes. Therefore, the Scutellaria alpina extract of the present invention can exhibit, by said effects, skin homeostasis maintenance and anti-stress effects, and thus can be widely used as a composition for maintaining skin homeostasis in the pharmaceutical or cosmetic field.

【FIG. 1】

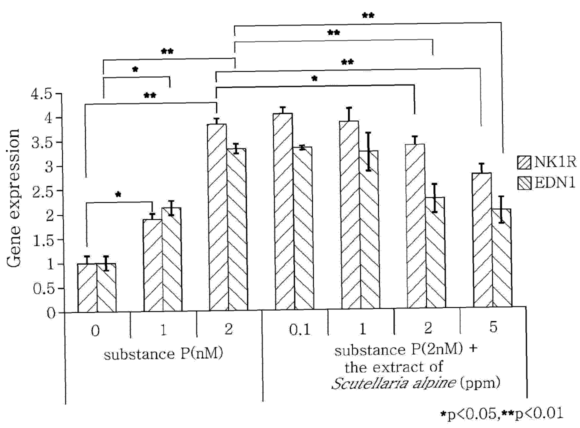


EP 3 165 231 A1

**Description**

[Technical Field]

The present disclosure relates to a composition comprising an alpine skullcap extract.

[Background Art]

Stress, which is called the cause of all modern diseases, refers to a nonspecific general adaptation syndrome as a response to a threat to the body which occurs when a stimulus of a certain degree or higher acts on the body in an injurious manner, regardless of the kind of the stimulus (Selye, H.: The stress of life, Toronto, Longnans Green and Co., pp. 1-50, 1958). The causes of stress comprise largely (1) physical factors: overwork, insufficient sleep, trauma, intoxication, etc.; (2) external factors: natural environment, cultural situation, interpersonal relation in family or workplace, etc.; and (3) internal factors: personality, psychological conflict in life, mental trauma experienced in infancy or childhood, etc.

When stress persists for a long time, it may cause physical diseases such as heart disease, gastric ulcer and hypertension or psychological maladjustment such as insomnia, neurosis and depression. Also, it may cause other diseases because the physiological activity and immune activity of the body decrease and the homoeostasis of the body is negatively affected. In the modern society full of conflicts and competitions, most people are physically/mentally stressed more because they have to adapt to the society which changes structurally or functionally in a complicated manner. The decrease in immune activity caused by stress may lead to disruption of skin homeostasis, which in turn may weaken the skin moisturizing and barrier functions of protecting the skin from harmful external factors.

**[Disclosure]**

[Technical Problem]

The present disclosure is directed to providing a composition having a superior effect of maintaining skin homeostasis.

[Technical Solution]

In an aspect, the present disclosure provides a composition for maintaining skin homeostasis, which comprises an extract of alpine skullcap (*Scutellaria alpina*) belonging to the genus *Scutellaria* as an active ingredient.

[Advantageous Effects]

A composition for maintaining skin homeostasis of the present disclosure, which comprises an alpine skullcap (*Scutellaria alpina)* extract as an active ingredient, exhibits an effect of reducing the expression of NK1R (neurokinin 1 receptor) and EDN1 (endothelin 1) genes as well as an anti-stress effect against substance P. Accordingly, due to such effects, the composition of the present disclosure can exhibit benefits for maintaining skin homeostasis as well as an anti-stress effect, and thus can be widely used in formulations for external application to skin, pharmaceutical compositions and cosmetic compositions because it is safe with no negative effect on cells.

[Brief Description of Drawings]

FIG. 1 shows a result of analyzing the effect of an alpine skullcap (*Scutellaria alpina)* extract according to an aspect of the present disclosure on the expression of NK1R and EDN1 genes.

FIG. 2 shows a result of analyzing the effect of an alpine skullcap (*Scutellaria alpina)* extract according to an aspect of the present disclosure on the amount of EDN1.

[Best Mode]

Korean Patent Application No. 10-2014-0052274, filed on April 30, 2014, is incorporated in the present disclosure in its entirety. Also, this application claims the priority of Korean Patent Application No. 10-2014-0052274, the contents of which in its entirety is incorporated herein by reference.

Hereinafter, specific embodiments of the present disclosure are described in detail such that those of ordinary skill in the art to which the present disclosure belongs can easily carry out the present disclosure.

In an aspect of the present disclosure, the neuropeptide substance P is known to be associated with the regulation of mood disorder, anxiety, stress, pain, vomiting, etc. Therefore, if the factors induced by the substance P can be controlled, it may be possible to prevent the decline in skin homeostasis caused by stress-induced decrease of immune activity. In an aspect of the present disclosure, the substance P may have the following amino acid sequence and the following chemical formula. Amino acid sequence of substance P: Arg Pro Lys Pro Gin Gin Phe Phe Gly Leu Met (RPKPQQFFGLM).

[Chemical formula]

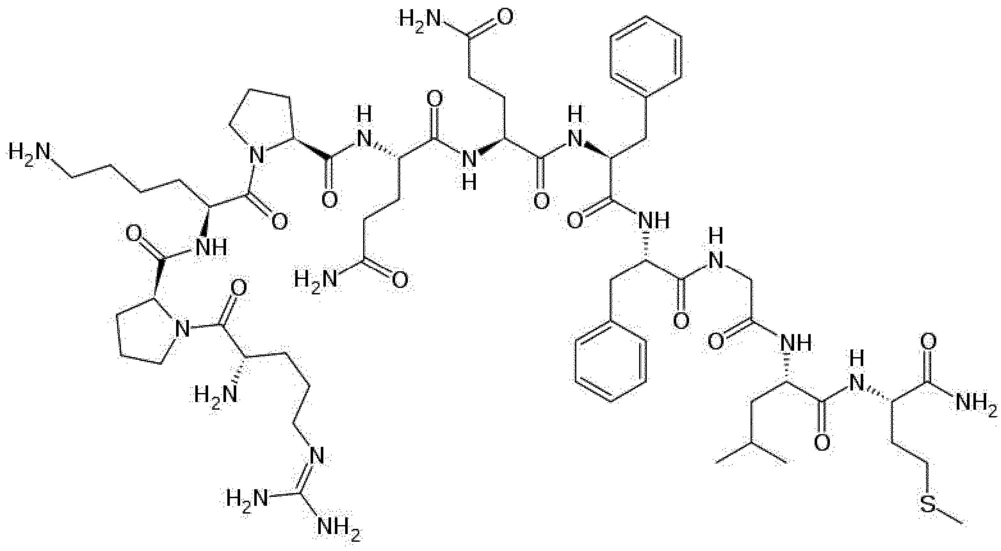

It is known that increased concentration of substance P leads to increased expression of its receptor NK1R (neurokinin 1 receptor, also known as substance P receptor (SPR) or tachykinin receptor 1 (TACR1)) and its protein. The signaling of NK1 R stimulates ECE1 (endothelin converting enzyme 1) and thereby increases the expression of activated EDN1 (endothelin 1) and its protein. Because substance P increases the expression of NK1R and EDN1 genes, a substance capable of inhibiting the expression of the two genes is expected to inhibit the activity of substance P and to exhibit an effect of maintaining skin homeostasis and an anti-stress effect by suppressing physiological effects caused by stressors. In addition, increase in activated EDN1 in cells due to the NK1 R signaling may lead to increased melanin production by melanocytes. Accordingly, it is expected that, when the expression of EDN1 is inhibited, skin homoeostasis can be maintained because melanin production in cells can be inhibited and suppressed and skin diseases caused by melanin production such as pigmentation can be treated, improved or prevented.

In an aspect, the present disclosure may relate to a composition which comprises an alpine skullcap (*Scutellaria alpina)* extract as an active ingredient. Specifically, in an aspect of the present disclosure, the composition may be a composition for maintaining skin homeostasis.

The composition according to an aspect of the present disclosure may be a pharmaceutical, food or cosmetic composition. Specifically, in an aspect of the present disclosure, the composition according to the present disclosure may be a composition for extremal application to skin.

In an aspect of the present disclosure, the term "skin homeostasis" may mean that the skin's environment or physiological condition such as pH, temperature, blood flow, a water content, etc. is maintained continuously as a normal state.

In an aspect, the present disclosure may relate to a method for maintaining skin homeostasis, which comprises administering an alpine skullcap (*Scutellaria alpina)* extract to a subject in need of maintenance of skin homeostasis. Specifically, the administration may be achieved by an administration method described in the present disclosure.

In an aspect, the present disclosure may relate to a use of an alpine skullcap (*Scutellaria alpina)* extract for maintenance of skin homeostasis.

In an aspect, the present disclosure may relate to an alpine skullcap (*Scutellaria alpina)* extract for use in maintenance of skin homeostasis.

In an aspect, the present disclosure may relate to composition for fighting against stress or a composition for

reducing stressors, which comprises an alpine skullcap (*Scutellaria alpina)* extract as an active ingredient. Specifically, in an aspect of the present disclosure, the stressor may be substance P.

In an aspect, the present disclosure may relate to a method for reducing stressors, which comprises administering an alpine skullcap (*Scutellaria alpina*) extract to a subject in need of reduction of stressors. Specifically, the administration may be achieved by an administration method described in the present disclosure.

In an aspect, the present disclosure may relate to a use of an alpine skullcap *(Scutellaria alpina)* extract for reducing stressors.

In an aspect, the present disclosure may relate to an alpine skullcap *(Scutellaria alpina)* extract for use in reducing stressors.

In an aspect, the present disclosure may relate to a composition for enhancing skin immune function, skin moisturizing function or skin barrier function, which comprises an alpine skullcap (*Scutellaria alpina)* extract as an active ingredient.

In an aspect, the present disclosure may relate to a method for enhancing skin immune function, skin moisturizing function or skin barrier function, which comprises administering an alpine skullcap *(Scutellaria alpina)* extract to a subject in need of enhancement of skin immune function, skin moisturizing function or skin barrier function. Specifically, the administration may be achieved by an administration method described in the present disclosure.

In an aspect, the present disclosure may relate to a use of an alpine skullcap (*Scutellaria alpina)* extract for enhancing skin immune function, skin moisturizing function or skin barrier function.

In an aspect, the present disclosure may relate to an alpine skullcap (*Scutellaria alpina)* extract for use in enhancing skin immune function, skin moisturizing function or skin barrier function.

In an aspect, the present disclosure may relate to composition for inhibiting the activity of the neuropeptide substance P, which comprises an alpine skullcap (*Scutellaria alpina)* extract as an active ingredient.

In an aspect, the present disclosure may relate to a method for inhibiting the activity of the neuropeptide substance P, which comprises administering an alpine skullcap (*Scutellaria alpina)* extract to a subject in need of inhibition of the activity of the neuropeptide substance P. Specifically, the administration may be achieved by an administration method described in the present disclosure.

In an aspect, the present disclosure may relate to a use of an alpine skullcap (*Scutellaria alpina)* extract for inhibiting the activity of the neuropeptide substance P.

In an aspect, the present disclosure may relate to an alpine skullcap (*Scutellaria alpina)* extract for use in inhibiting the activity of the neuropeptide substance P.

In an aspect, the present disclosure may relate to a composition for inhibiting the expression of the NK1R (neurokinin 1 receptor, also known as substance P receptor (SPR) or tachykinin receptor 1 (TACR1)) (ACCESSION: NM_001058) gene or the EDN1 (endothelin 1) (ACCESSION: NM_001168319) gene or for reducing the amount of EDN1, which comprises an alpine skullcap (*Scutellaria alpina)* extract as an active ingredient.

In an aspect, the present disclosure may relate to a method for inhibiting the expression of the NK1R gene or the EDN1 (endothelin 1) gene or reduction of the amount of EDN1, which comprises administering an alpine skullcap (*Scutellaria alpina)* extract to a subject in need of inhibition of the inhibition of the NK1 R gene or the EDN1 gene or reduction of the amount of EDN1. Specifically, the administration may be achieved by an administration method described in the present disclosure.

In an aspect, the present disclosure may relate to use of an alpine skullcap (*Scutellaria alpina)* extract for inhibiting the expression of the NK1R gene or the EDN1 (endothelin 1) gene or reducing the amount of EDN1.

In an aspect, the present disclosure may relate to an alpine skullcap (*Scutellaria alpina)* extract for use in inhibiting the expression of the NK1 R gene or the EDN1 (endothelin 1) gene or reducing the amount of EDN1.

In an aspect of the present disclosure, the concentration of the alpine skullcap (*Scutellaria alpina)* extract in the composition comprising the same may be 0.1-10 ppm based on the total volume of the composition, although not being limited thereto. Specifically, in an aspect of the present disclosure, the concentration of the alpine skullcap (*Scutellaria alpina)* extract in the composition comprising the same may be 0.01 ppm or higher, 0.1 ppm or higher, 0.5 ppm or higher, 0.6 ppm or higher, 0.7 ppm or higher, 0.8 ppm or higher, 0.9 ppm or higher, 1.0 ppm or higher, 1.3 ppm or higher, 1.5 ppm or higher, 1.7 ppm or higher, 2.0 ppm or higher, 2.3 ppm or higher, 2.5 ppm or higher, 2.7 ppm or higher, 3.0 ppm or higher, 3.5 ppm or higher, 4.0 ppm or higher, 4.1 ppm or higher, 4.2 ppm or higher, 4.3 ppm or higher, 4.4 ppm or higher, 4.5 ppm or higher, 4.6 ppm or higher, 4.7 ppm or higher, 4.8 ppm or higher, 4.9 ppm or higher, 5 ppm or higher, 5.2 ppm or higher, 5.4 ppm or higher, 5.6 ppm or higher, 5.8 ppm or higher, 6.0 ppm or higher, 6.5 ppm or higher, 7.0 ppm or higher, 7.5 ppm or higher, 8.0 ppm or higher, 9.0 ppm or higher, 10.0 ppm or higher, 11.0 ppm or higher, 12.0 ppm or higher, 50 ppm or higher, 100 ppm or higher, 500 ppm or higher or 1000 ppm or higher, although not being limited thereto, and may be 2000 ppm or lower, 1000 ppm or lower, 500 ppm or lower, 100 ppm or lower, 50 ppm or lower, 40 ppm or lower, 30 ppm or lower, 20 ppm or lower, 15.0 ppm or lower, 10.0 ppm or lower, 8.0 ppm or lower, 6.0 ppm or lower, 5.8 ppm or lower, 5.6 ppm or lower, 5.4 ppm or lower, 5.2 ppm or lower, 5.0 ppm or lower, 4.8 ppm or lower, 4.6 ppm or lower, 4.4 ppm or lower, 4.2 ppm or lower, 4.0 ppm or lower, 3.7 ppm or lower, 3.5 ppm or lower, 3.3

ppm or lower, 3.0 ppm or lower, 2.8 ppm or lower, 2.6 ppm or lower, 2.4 ppm or lower, 2.2 ppm or lower, 2.0 ppm or lower, 1.8 ppm or lower, 1.6 ppm or lower, 1.4 ppm or lower, 1.2 ppm or lower, 1.0 ppm or lower, 0.8 ppm or lower, 0.6 ppm or lower, 0.3 ppm or lower or 0.1 ppm or lower, based on the total volume of the composition.

In an aspect of the present disclosure, the alpine skullcap (*Scutellaria alpina*) may be one or more selected from a group consisting of a leaf, a fruit, a flower, a stem and a root of a plant alpine skullcap (*Scutellaria alpina).* Specifically, in an aspect of the present disclosure, the alpine skullcap (*Scutellaria alpina)* may be a mixture of the flower, leaf and stem of the plant alpine skullcap (*Scutellaria alpina*).

In an aspect of the present disclosure, the alpine skullcap (*Scutellaria alpina*) extract may be an extract of one or more of a leaf, a fruit, a flower, a stem and a root of a plant alpine skullcap (*Scutellaria alpina)* and also may be a mixture of these extracts.

In an aspect of the present disclosure, the alpine skullcap (*Scutellaria alpina*) extract may be prepared by a method comprising (1) a step of extracting alpine skullcap (*Scutellaria alpina)* with a water, an organic solvent or a combination thereof.

In an aspect of the present disclosure, the method may furthercomprise, prior to the step (1), a step of processing alpine skullcap (*Scutellaria alpina).* Specifically, the processing may be drying and pulverizing alpine skullcap (*Scutellaria alpina*). However, without being limited thereto, any processing for facilitating the extraction is comprised. Specifically, the drying may be sunlight drying, hot air drying, evaporation drying, spray drying or freeze drying, more specifically hot air drying. On the other hand, in an aspect of the present disclosure, live alpine skullcap (*Scutellaria alpina)* may be extracted as it is without any processing.

In an aspect of the present disclosure, the method may further comprise a step of removing the solvent by distillation following the extraction. Specifically, the distillation may be vacuum distillation.

In an aspect of the present disclosure, the method may further comprise a step of adding one or more of glycerin and a preservative to the resulting concentrate following the distillation.

In an aspect of the present disclosure, the method may further comprise a step of filtering following the solvent removal or following the addition of one or more of glycerin and a preservative.

In an aspect of the present disclosure, the alpine skullcap *(Scutellaria alpina*) extract may be an extract of one or more selected from a group consisting of a water, an organic solvent and a mixture thereof. Specifically, in an aspect of the present disclosure, the organic solvent may be one or more selected from a group consisting of a $C_1$-$C_6$ lower alcohol, butylene glycol and propylene glycol. More specifically, the lower alcohol may be ethanol.

The melanocytes used in the present disclosure may be the melanocytes disclosed in PCT/KR2012/007815 or Korean Patent Application No. 10-2011-0099728 (keratinocyte-adapted melanocytes, KaMC). The melanocytes used in the present disclosure may be those obtained according to the method and procedure disclosed in the references and may also be cultured according to the method disclosed in the references. The melanocytes were deposited in the Korean Collection for Type Culture (KCTC) of Korea Research Institute of Bioscience and Biotechnology on Sep. 14, 2011 under the Budapest Treaty and were given the accession number KCTC 12015BP.

In the present disclosure, the "skin" refers to the organ covering the surface of an organism. It is composed of the epidermis, the dermis and the subcutaneous fat layer and is used in the broadest concept, comprising not only the tissues covering the face or the entire outer part of the body but also the scalp and hair.

In the present disclosure, the "alpine skullcap (*Scutellaria alpina)"* refers to a plant belonging to the genus *Scutellaria* of the family *Lamiaceae.* It can reach a height of about 10-30 cm. The stems are hairy, square and prostrate-ascending. The flowers are blue-violet or purple, 2.4-3 cm long. Alpine skullcap prefers rocky areas in high calcareous mountains at elevations of 1400-2500 m.

In the present disclosure, the "extract" refers to a substance extracted from a natural product, regardless of the extraction method, extraction solvent, extracted components or the type of the extract. It is used in a broad concept, comprising any substance that can be obtained by processing or treatment of a substance extracted from a natural product. Specifically, the processing or treatment may be fermenting or enzymatically treating the extract. Accordingly, in the present disclosure, the extract is used in a broad concept, comprising a fermentation product, a concentrate and a dried product. Specifically, in the present disclosure, the extract may be a fermentation product.

In the present disclosure, the "alpine skullcap (*Scutellaria alpina)* extract" comprises any substance obtained by extracting alpine skullcap (*Scutellaria alpina*)*,* regardless of the extraction method, extraction solvent, extracted components or the type of the extract. It comprises a substance obtained by an extraction method comprising a process treating with heat, an acid, a base, an enzyme, etc. and is used in a broad concept, comprising any substance that can be obtained by processing or treatment of a substance extracted from alpine skullcap (*Scutellaria alpina*). Specifically, the processing or treatment may be fermenting or enzymatically treating the alpine skullcap (*Scutellaria alpina)* extract. Accordingly, the alpine skullcap (*Scutellaria alpina)* extract of the present disclosure may be a fermentation product.

In an aspect of the present disclosure, the "alpine skullcap (*Scutellaria alpina*)" may be an extract, live alpine skullcap (*Scutellaria alpina),* a pulverization product of live alpine skullcap (*Scutellaria alpina),* a dried product of live alpine skullcap (*Scutellaria alpina),* a dried pulverization product of live alpine skullcap (*Scutellaria alpina)* or a fermentation product of alpine skullcap (*Scutellaria alpina),* although not being limited thereto. And, the alpine skullcap (*Scutellaria alpina)* used in the present disclosure is not limited in the way how it is obtained. It may be either cultivated or purchased commercially. Also, all or part of its areal part or root part may be used. More specifically, one or more selected from a group consisting of a leaf, a stem, a root and a flower of a plant alpine skullcap (*Scutellaria alpina)* may be used. More specifically, the flower, leaf and stem may be used. The alpine skullcap (*Scutellaria alpina)* of the present disclosure is not necessarily dried and may be in any form as long as the active ingredients of alpine skullcap (*Scutellaria alpina*) can be adequately extracted.

In an aspect of the present disclosure, the water comprises distilled water or purified water, and the organic solvent comprises one or more selected from a group consisting of an alcohol such as a $C_1$-$C_5$ lower alcohol, acetone, ether, ethyl acetate, diethyl ether, methyl ethyl ketone and chloroform, although not being limited thereto.

In an aspect of the present disclosure, the alpine skullcap (*Scutellaria alpina*) extract may comprise a $C_1$-$C_6$ alcohol extract of alpine skullcap (*Scutellaria alpina*). Specifically, the alcohol may be methanol or ethanol.

In an aspect of the present disclosure, the alpine skullcap (*Scutellaria alpina*) extract may be obtained by a method comprising a step of extracting alpine skullcap (*Scutellaria alpina)* with a water, an organic solvent or a mixture thereof.

In an aspect of the present disclosure, the alpine skullcap (*Scutellaria alpina*) extract may be a crude extract of a solvent selected from a group consisting of a water, an organic solvent and a combination thereof. The organic solvent may be a $C_1$-$C_6$ alcohol. Specifically, the $C_1$-$C_6$ alcohol may be methanol or ethanol. In an aspect of the present disclosure, when the alpine skullcap (*Scutellaria alpina)* is extracted with a solvent, the solvent may be added in an amount of about 5-15 times that of the alpine skullcap (*Scutellaria alpina).* Specifically, solvent may be added in an amount of about 10 times that of the alpine skullcap (*Scutellaria alpina),* although not being limited thereto.

In an aspect of the present disclosure, the extraction may be performed by hot water extraction, ethanol extraction, heating extraction, cold extraction, reflux extraction, reflux condensation extraction, ultrasonic extraction, etc. Any extraction method known to those skilled in the art may be used without limitation. Specifically, the extraction may be performed by hot water extraction or ethanol extraction.

In an aspect of the present disclosure, the extraction may be performed at room temperature. However, for more effective extraction, it may be performed at elevated temperatures. The extraction may be performed specifically at about 40-100 °C, more specifically at about 80 °C, although not being limited thereto. The extraction may be performed for about 2-14 hours, specifically for about 8-14 hours, more specifically for about 11-13 hours, most specifically for 12 hours. However, the extraction time is not limited thereto and may vary depending on such conditions as extraction solvent, extraction temperature, etc. The extraction may be performed more than once in order to obtain a larger amount of the active ingredients. The extraction may be performed continuously specifically 1-5 times, more specifically 3 times, and the resulting extracts may be combined for further use.

In an aspect of the present disclosure, the alpine skullcap *(Scutellaria alpina*) extract may comprise the crude extract of alpine skullcap (*Scutellaria alpina)* as described above and may also comprise a soluble fraction of an organic solvent with low polarity, which is obtained by further extracting the crude extract with the organic solvent. In an aspect of the present disclosure, the organic solvent may be hexane, methylene chloride, ethyl acetate, n-butanol, etc., although not being limited thereto. The extract or the soluble fraction of the extract may be used as it is, after being filtered and then concentrated, or after being concentrated and dried.

In an aspect of the present disclosure, the drying may be evaporation drying, spray drying or freeze drying. Specifically, freeze drying may be performed at -50 to -70 °C for 3-4 days.

Formulations of the cosmetic composition according to an aspect the present disclosure are not particularly limited but may be selected properly depending on purposes. For example, the composition may be prepared into one or more formulation selected from a group consisting of a skin lotion, a skin softener, a skin toner, an astringent lotion, a milk lotion, a moisturizing lotion, a nourishing lotion, a massage cream, a nourishing cream, a moisturizing cream, a hand cream, a foundation, an essence, a nourishing essence, a pack, a soap, a cleansing foam, a cleansing lotion, a cleansing cream, a body lotion and a body cleanser, although not being limited thereto.

When the cosmetic composition according to the present disclosure is formulated as a paste, a cream or a gel, an animal fiber, a plant fiber, wax, paraffin, starch, tragacanth, a cellulose derivative, polyethylene glycol, silicone, bentonite, silica, talc, zinc oxide, etc. may be used as a carrier component.

When the cosmetic composition according to the present disclosure is formulated as a powder or a spray, lactose, talc, silica, aluminum hydroxide, calcium silicate or polyamide powder may be used as a carrier component. In particular, when it is formulated as a spray, it may further comprise a propellant such as chlorofluorohydrocarbon, propane/butane or dimethyl ether.

When the cosmetic composition according to the present disclosure is formulated as a solution or an emulsion, a solvent, a solubilizer or an emulsifier may be used as a carrier component. For example, water, ethanol, isopropanol, ethyl carbonate, ethyl acetate, benzyl alcohol, benzyl benzoate, propylene glycol, 1,3-butyl glycol oil, glycerol fatty ester, or fatty acid ester of polyethylene glycol or sorbitan may be used.

When the cosmetic composition according to the present disclosure is formulated as a suspension, a diluent such as water, ethanol or propylene glycol, a suspending agent such as ethoxylated isostearyl alcohol, polyoxyethylene sorbitol ester and polyoxyethylene sorbitan ester, microcrystalline cellulose, aluminum metahydroxide, bentonite, agar, tragacanth, etc. may be used as a carrier component.

When the cosmetic composition according to the present disclosure is formulated as a surfactant-comprising cleanser, fatty alcohol sulfate, fatty alcohol ether sulfate, sulfosuccinic acid monoester, isethionate, an imidazolinium derivative, methyl taurate, sarcosinate, a fatty acid amide ether sulfate, an alkyl amidobetaine, a fatty alcohol, a fatty acid glyceride, a fatty acid diethanolamide, a vegetable oil, a lanolin derivative, an ethoxylated glycerol fatty acid ester, etc. may be used as a carrier component.

The cosmetic composition according to the present disclosure may further comprise a functional additive and ingredients commonly used in a cosmetic composition, in addition to the alpine skullcap (*Scutellaria* alpina) extract. The functional additive may comprise those selected from a group consisting of a water-soluble vitamin, an oil-soluble vitamin, a polypeptide, a polysaccharide, a sphingolipid and a seaweed extract.

If necessary, the cosmetic composition of the present disclosure may further comprise, in addition to the functional additive, the ingredients commonly used in a cosmetic composition. Examples of the further comprised ingredients may comprise an oil, a fat, a humectant, an emollient, a surfactant, an organic or inorganic pigment, an organic powder, a UV absorbent, a preservative, a sterilizer, an antioxidant, a plant extract, a pH control agent, an alcohol, a colorant, a flavor, a blood circulation promoter, a cooling agent, an antiperspirant, purified water, etc.

The present disclosure also relates to a composition for external application to skin, which comprises an alpine skullcap (*Scutellaria alpina)* extract as an active ingredient. The composition for external application to skin refers to any type of composition that can be applied from outside the skin and various types of cosmetics may be comprised therein.

The pharmaceutical composition according to the present disclosure may be in the form of various oral or parenteral formulations. When the composition is formulated, a commonly used diluent or excipient such as a filler, an extender, a binder, a wetting agent, a disintegrant, a surfactant, etc. is used. Solid formulations for oral administration comprise a tablet, a pill, a powder, a granule, a soft or hard capsule, etc., and these solid formulations may be prepared by mixing with at least one excipient such as starch, calcium carbonate, sucrose, lactose, gelatin, etc. In addition to the simple excipient, a lubricant such as magnesium stearate, talc, etc. may also be used. Examples of liquid formulations for oral administration comprise a suspension, a liquid for internal use, an emulsion, a syrup, etc. In addition to a commonly used simple diluent such as water and liquid paraffin, various excipients such as a humectant, a sweetener, an aromatic, a preservative, etc. may also be comprised. Formulations for parenteral administration comprise a sterilized aqueous solution, a non-aqueous solution, a suspension, an emulsion, a lyophilized product and a suppository. The non-aqueous solution or suspension may comprise propylene glycol, polyethylene glycol, a vegetable oil such as olive oil, an injectable ester such as ethyl oleate, etc. As a base of the suppository, witepsol, macrogol, tween 61, cocoa butter, laurin butter, glycerogelatin, etc. may be used.

The composition of the present disclosure may contain a pharmaceutically acceptable salt of the active ingredient, and may be used alone or in combination with another pharmaceutically active compound(s). The salt is not particularly limited as long as it is pharmaceutically acceptable. For example, a salt of hydrochloric acid, sulfuric acid, nitric acid, phosphoric acid, hydrofluoric acid, hydrobromic acid, formic acid, acetic acid, tartaric acid, lactic acid, citric acid, fumaric acid, maleic acid, succinic acid, methanesulfonic acid, benzenesulfonic acid, toluenesulfonic acid, naphthalenesulfonic acid, etc. may be used.

The composition of the present disclosure may be administered parenterally or orally depending on the desired purposes. A daily dose of 0.1-500 mg, specifically 1-100 mg, per kg body weight may be administered once or several times a day. The administration dose for a particular patient may be changed depending on the body weight, age, sex and health conditions of the patient, diet, administration time, administration method, rate of excretion, severity of a disease, and so forth.

The pharmaceutical composition according to the present disclosure may be formulated into any pharmaceutically appropriate form comprising oral formulations such as a powder, a granule, a tablet, a soft or hard capsule, a suspension, an emulsion, a syrup, an aerosol, etc., formulations for external application to skin such as an ointment, a cream, etc., a suppository, an injection, a sterile solution for injection, etc. according to commonly employed methods. Specifically, it may be formulated into an injection or a formulation for external application to skin.

The composition according to the present disclosure may be administered to mammals comprising rat, mouse, cattle, human, etc. via various routes comprising parenteral and oral routes. All types of administration may be expected. For example, it may be administered orally, transdermally, rectally, intravenously, intramuscularly, subcutaneously, intrauterinally or intracerebroventricularly.

The composition according to the present disclosure may be administered via various routes that can be easily selected by those skilled in the art. In particular, the pharmaceutical composition according to the present disclosure may be applied on skin as a formulation for external application to skin.

In an aspect of the present disclosure, the food composition may be a health functional food composition.

Formulations of the food composition according to the present disclosure are not particularly limited. For example, it may be formulated into a tablet, a granule, a powder, a liquid such as a drink, a caramel, a gel, a bar, etc. The food composition of each formulation may comprise, in addition to the active ingredient, ingredients commonly used in the art that can be selected by those skilled in the art without difficulty. In this case, a synergic effect may be achieved.

In the food composition according to the present disclosure, determination of the administration dose of the active ingredient is within the level of those skilled in the art. For example, a daily dosage may be 0.1-5000 mg/kg/day, more specifically, 50-500 mg/kg/day. However, without being limited thereto, the administration dose may vary depending on various factors such as the age and health conditions of the subject, presence of complication(s), etc.

For example, the food composition according to the present disclosure may be various foods such as a chewing gum, a caramel, a candy, a popsicle, confectionery, etc., drinks such as a soft drink, a mineral water, an alcohol beverage, etc. or health functional foods comprising vitamin, mineral, etc.

In addition, the food composition according to the present disclosure may comprise various nutrients, vitamins, minerals (electrolytes), flavors comprising synthetic and natural flavors, colorants, extenders (cheese, chocolate, etc.), pectic acid and salts thereof, alginic acid and salts thereof, organic acids, protective colloidal thickeners, pH control agents, stabilizers, preservatives, glycerin, alcohols, carbonating agents used in carbonated drinks, etc. And, the functional food composition of the present disclosure may comprise pulps used to prepare natural fruit juice, fruit juice drinks and vegetable drinks. These ingredients may be used independently or in combination. The content of these additives is of no great importance. Usually, they are comprised within a range of about 0-20 parts by weight based on 100 parts by weight of the composition of the present disclosure.

Hereinafter, the present disclosure will be described in detail through examples and test examples. However, the following examples and test examples are for illustrative purposes only and the scope of the present disclosure is not limited by the examples and test examples.

[Example 1] Preparation of alpine skullcap *(Scutellaria alpina)* extract

The flower, leaf and stem of alpine skullcap (*Scutellaria alpina)* were harvested, hot air dried and pulverized into powder. The resulting powder was extracted with an ethanol/water solution and then the ethanol was removed through vacuum distillation. After adding glycerin and a preservative to the resulting concentrate, an alpine skullcap (*Scutellaria alpina)* extract was obtained by filtration. The alpine skullcap (*Scutellaria alpina)* extract (ALPAFLOR® SCUTELLARIA AO) prepared according to the above-described method was purchased from DSM Nutritional Products Ltd. (4002 Basel, Switzerland) for use in the following test examples.

[Test Example 1] Gene expression assay

(1) Cell culturing

Melanocytes (KaMC; KCTC 12015BP) deposited in the Korean Collection for Type Culture (KCTC) were cultured in M-254 medium (Gibco BRL, NY, USA) supplemented with Human Melanocyte Growth Supplement (HMGS; Gibco BRL, NY, USA) on a 6-well plate, while changing the medium every other day, in a 5% $CO_2$ incubator at 37 °C until the cells occupied 95% of the plate area (95% confluence).

(2) Treatment with extract

When the density of the melanocytes on the 6-well plate reached about 80% (~80% confluence), they were incubated for a total of 5 days with 2 nM of stress-inducing substance P (Bachem Fechemikalien, Bubendorf, Switzerland) or the alpine skullcap (*Scutellaria alpina)* extract of Example 1 of different concentrations (0, 0.1, 1, 2 and 5 ppm). The cells not treated with substance P or the alpine skullcap *(Scutellaria alpina)* extract of Example 1 were used as a control group.

(3) Extraction and quantification of RNA

After removing the medium form the melanocytes cultured on the 6-well plate, the cells were washed 2 times with cold phosphate-buffered saline (PBS) and the remaining water was completely removed. After adding 1 mL of TRIzol reagent (Invitrogen, CA, USA) to the cells and collecting all the cells, the cells were mixed well with 200 μL of chloroform and kept at room temperature for 5 minutes. Then, after centrifugation at 4 °C and 12,000 rpm for 15 minutes, the transparent supernatant was transferred to a fresh tube, mixed well with the same volume of isopropyl alcohol (Sigma-Aldrich, St. Louis, USA) and kept at room temperature for 10 minutes. After centrifuging again at 4 °C and 12,000 rpm for 10 minutes, the supernatant was discarded. After adding 1 mL of 100% EtOH (Sigma-Aldrich, St. Louis, USA) to the

remaining pellets and slightly washing, centrifugation was performed at 4 °C and 12,000 rpm for 5 minutes. After adding 1 mL of 70% EtOH again and washing, followed by centrifugation at 4 °C and 12,000 rpm for 5 minutes, the pellets were dried until they became transparent. RNA extraction was completed by adding 50 μL of triple distilled water to the dried pellets. 1 μL of the RNA was quantified using Synergy II (Biotek, USA).

(4) Synthesis of cDNA

For synthesis of cDNA, 1 μg of the quantified RNA and the RevertAid First Strand cDNA synthesis kit (Thermo Scientific, USA) were added to a 0.2-mL tube and cDNA was synthesized by reverse transcription-polymerase chain reaction (RT-PCR) using the C-1000 Thermo Cycler PCR machine (Bio-Rad, USA).

(5) Gene expression assay by real-time PCR

The synthesized cDNA was diluted to 1/20 using distilled water. After adding 10 μL of Taqman Gene Expression Master Mix (Life Technologies, USA) and 1 μL of Assays-on-Demand™ (Life Technologies, USA) of NK1 R (ACCESSION: NM_001058) and EDN1 (ACCESSION: NM_001168319) genes to 9 μL of the diluted cDNA, the expression of the genes was analyzed by relative quantification using 7500 Fast Real-Time PCR (Life Technologies, USA) with glyceraldehyde 3-phosphate dehydrogenase (GAPDH, ACCESSION: NM001256799) as a control gene. That is to say, the expression levels of the NK1 R and EDN1 genes were compared with that of GAPDH. The result is shown in FIG. 1.

From FIG. 1, it can be seen that the expression of the NK1 R and EDN1 genes was increased to 3.8 and 3.3, respectively, when treated with 2 nM substance P as compared to 1 of the control group. When the cells were treated with 2 nM substance P and then with 5 ppm of the alpine skullcap (*Scutellaria alpina)* extract according to an aspect of the present disclosure was added, the expression of the NK1R and EDN1 genes was decreased to 2.7 and 2.0, respectively. From this result, it was confirmed that the alpine skullcap (*Scutellaria alpina)* extract according to an aspect of the present disclosure is effective in reducing the biological phenomena induced by substance P. This suggests that it has an effect of restoring skin homeostasis that has been disrupted by stress as well as an anti-stress effect. In particular, considering that the NK1 R gene is a gene which expresses the receptor protein of substance P, it is expected that the decreased expression of the gene will lead to decreased uptake of substance P by the body and prevent disruption of skin homeostasis caused by stressors such as substance P.

[Test Example 2] Quantification of endothelin 1

(1) Recovery of cells and quantification of proteins

The melanocytes (KaMC) cultured on the 6-well plate were added to 400 μL of an Accutase solution (Millipore, CA, USA). After agitation for 1 minute, a total of 1 mL of the cell-comprising medium was recovered by adding 600 μL of M-254 medium. Then, after centrifugation at 12,000 rpm for 15 minutes, the supernatant was discarded and 20 μL of MCL lysis buffer (Sigma-Aldrich, St. Louis, USA) was added to the remaining pellets. After mixing well and centrifuging again at 12,000 rpm for 15 minutes, the supernatant and the cell debris were separated.

After adding 1 μL of the separated supernatant to a 96-well plate, distilled water was added to make the final volume 25 μL. Then, the quantity of proteins were determined at 562 nm using the BCA protein assay kit (Thermo Scientific, IL, USA). The result is shown in Table 1.

[Table 1]

| Sample | | Mean (μg/μL) | Standard deviation |
|---|---|---|---|
| Substance P (nM) | Alpine skullcap (*Scutellaria alpina*) extract (ppm) | | |
| 0 | 0 | 4.2159878 | 0.598475 |
| 1 | 0 | 4.002843 | 0.1514731 |
| 2 | 0 | 4.011147 | 0.2256984 |
| | 0.1 | 4.05168 | 0.195863 |
| | 1 | 4.0045458 | 0.22548151 |
| | 2 | 4.62816 | 0.38052156 |
| | 5 | 4.1199856 | 0.2411025 |

(2) Endothelin 1 ELISA assay

The culture medium of the cells treated with the extract in (2) of Test Example 1 were recovered and the amount of endothelin 1 (EDN1) comprised in the medium was measured at 450 nm using the human total endothelin ELISA kit (MyBioSource, USA). FIG. 2 shows a result of calculating the proportion of the protein in total proteins according to Equation 1.

[Equation 1]

$$\text{Endothelin content (\%)} = (\text{Absorbance} / \text{Total protein}) \times 100$$

As seen from FIG. 2, when the cells were treated with 2 nM substance P, the content of endothelin 1 was increased to 124% P as compared to 100% of the control group. When the cells were treated with 2 nM substance P and then 5 ppm of the alpine skullcap (*Scutellaria alpina)* extract according to an aspect of the present disclosure was added, the content of endothelin 1 was decreased to 100%, comparable to that of the control group. This result shows that the alpine skullcap (*Scutellaria alpina)* extract according to an aspect of the present disclosure has a remarkable effect of decreasing the amount of endothelin 1. The decrease in the amount of endothelin 1 induced by substance P suggests that the alpine skullcap (*Scutellaria alpina)* extract according to an aspect of the present disclosure provides a remarkable effect of maintaining skin homeostasis as well as an anti-stress effect by suppressing the physiological effect caused by stressors.

Hereinafter, formulation examples of the composition according to an aspect of the present disclosure will be described. However, other formulations are also possible and the scope of the present disclosure is not limited by the formulation examples.

[Formulation Example 1] Soft capsule

8 mg of the alpine skullcap (*Scutellaria alpina)* extract of Example 1, 9 mg of vitamin E, 9 mg of vitamin C, 2 mg of palm oil, 8 mg of hydrogenated vegetable oil, 4 mg of yellow beeswax and 9 mg of lecithin were mixed according to a commonly employed method and a soft capsule was prepared by filling 400 mg of the mixture per capsule. Separately from this, a soft capsule sheet was prepared from 66 parts by weight of gelatin, 24 parts by weight of glycerin and 10 parts by weight of sorbitol, which was then filled with the mixture to prepare a soft capsule in which 400 mg of the composition according to the present disclosure is comprised.

[Formulation Example 2] Tablet

8 mg of the alpine skullcap (*Scutellaria alpina)* extract of Example 1, 9 mg of vitamin E, 9 mg of vitamin C, 200 mg of galactooligosaccharide, 60 mg of lactose and 140 mg of maltose were mixed and granulated using a fluidized-bed dryer. Then, after adding 6 mg of sugar ester, the mixture was prepared into a tablet comprising 500 mg of the composition according to a commonly employed method.

[Formulation Example 3] Drink

8 mg of the alpine skullcap (*Scutellaria alpina)* extract of Example 1, 9 mg of vitamin E, 9 mg of vitamin C, 10 g of glucose, 0.6 g of citric acid and 25 g of oligosaccharide syrup were mixed and 300 mL of purified water was added. 200 mL of the mixture was filled in a bottle. Then, a drink was prepared by sterilizing at 130 °C for 4-5 seconds.

[Formulation Example 4] Granule

8 mg of the alpine skullcap (*Scutellaria alpina)* extract of Example 1, 9 mg of vitamin E, 9 mg of vitamin C, 250 mg of anhydrous crystalline glucose and 550 mg of starch were mixed and granulated using a fluidized-bed granulator. The resulting granule was filled in a pouch.

[Formulation Example 5] Injection

An injection was prepared according to a commonly employed method with the composition described in Table 2.

[Table 2]

| Ingredient | Content |
| --- | --- |
| Alpine skullcap (*Scutellaria alpina*) extract of Example 1 | 10-50 mg |
| Sterilized distilled water for injection | adequate |
| pH control agent | adequate |

[Formulation Example 6] Health functional food

A health functional food was prepared according to a commonly employed method with the composition described in Table 3.

[Table 3]

| Ingredient | Content |
| --- | --- |
| Alpine skullcap (*Scutellaria alpina*) extract of Example 1 | 20 mg |
| Vitamin A acetate | 70 μg |
| Vitamin E | 1.0 mg |
| Vitamin $B_1$ | 0.13 mg |
| Vitamin $B_2$ | 0.15 mg |
| Vitamin $B_6$ | 0.5 mg |
| Vitamin $B_{12}$ | 0.2 μg |
| Vitamin C | 10 mg |
| Biotin | 10 μg |
| Nicotinamide | 1.7 mg |
| Folic acid | 50 μg |
| Calcium pantothenate | 0.5 mg |
| Ferrous sulfate | 1.75 mg |
| Zinc oxide | 0.82 mg |
| Magnesium carbonate | 25.3 mg |
| Potassium dihydrogen phosphate | 15 mg |
| Calcium monohydrogen phosphate | 55 mg |
| Potassium citrate | 90 mg |
| Calcium carbonate | 100 mg |
| Magnesium chloride | 24.8 mg |

Although specific compositions of vitamin and mineral mixtures suitable for a health functional food were described as above, the compositions may be changed as desired.

[Formulation Example 7] Health drink

A health drink was prepared according to a commonly employed method with the composition described in Table 4.

[Table 4]

| Ingredient | Content |
|---|---|
| Alpine skullcap (*Scutellaria alpina*) of Example 1 | 1000 mg |
| Citric acid | 1000 mg |
| Oligosaccharide | 100 g |
| Taurine | 1 g |
| Purified water | balance |

According to a commonly employed health drink preparation method, the above-described ingredients were mixed and heated at 85 °C for about 1 hour under agitation. The resulting solution was filtered and sterilized.

[Formulation Example 8] Softening lotion (skin lotion)

A softening lotion was prepared according to a commonly employed method with the composition described in Table 5.

[Table 5]

| Ingredient | Content (wt%) |
|---|---|
| Alpine skullcap (*Scutellaria alpina*) extract of Example 1 | 0.2 |
| Glycerin | 3.0 |
| Butylene glycol | 2.0 |
| Propylene glycol | 2.0 |
| Carboxyvinyl polymer | 0.1 |
| PEG-12 nonyl phenyl ether | 0.2 |
| Polysorbate 80 | 0.4 |
| Ethanol | 10.0 |
| Triethanolamine | 0.1 |
| Preservative, pigment and flavor | adequate |
| Purified water | balance |

[Formulation Example 9] Nourishing lotion (milk lotion)

A nourishing lotion was prepared according to a commonly employed method with the composition described in Table 6.

[Table 6]

| Ingredient | Content (wt%) |
|---|---|
| Alpine skullcap (*Scutellaria alpina*) extract of Example 1 | 1.0 |
| Glycerin | 3.0 |
| Butylene glycol | 3.0 |
| Propylene glycol | 3.0 |
| Carboxyvinyl polymer | 0.1 |
| Beeswax | 4.0 |
| Polysorbate 60 | 1.5 |
| Caprylic/capric triglyceride | 5.0 |

(continued)

| Ingredient | Content (wt%) |
|---|---|
| Squalane | 5.0 |
| Sorbitan sesquioleate | 1.5 |
| Liquid paraffin | 0.5 |
| Cetearyl alcohol | 1.0 |
| Triethanolamine | 0.2 |
| Preservative, pigment and flavor | adequate |
| Purified water | balance |

[Formulation Example 10] Nourishing cream

A nourishing cream was prepared according to a commonly employed method with the composition described in Table 7.

[Table 7]

| Ingredient | Content (wt%) |
|---|---|
| Alpine skullcap (*Scutellaria alpina*) extract of Example 1 | 2.0 |
| Glycerin | 3.0 |
| Butylene glycol | 3.0 |
| Liquid paraffin | 7.0 |
| β-Glucan | 7.0 |
| Carbomer | 0.1 |
| Caprylic/capric triglyceride | 3.0 |
| Squalane | 5.0 |
| Cetearyl glucoside | 1.5 |
| Sorbitan stearate | 0.4 |
| Polysorbate 60 | 1.2 |
| Triethanolamine | 0.1 |
| Preservative, pigment and flavor | adequate |
| Purified water | balance |

[Formulation Example 11] Massage cream

A massage cream was prepared according to a commonly employed method with the composition described in Table 8.

[Table 8]

| Ingredient | Content (wt%) |
|---|---|
| Alpine skullcap (*Scutellaria alpina*) extract of Example 1 | 2.0 |
| Glycerin | 8.0 |
| Butylene glycol | 4.0 |
| Liquid paraffin | 45.0 |

(continued)

| Ingredient | Content (wt%) |
|---|---|
| β-Glucan | 7.0 |
| Carbomer | 0.1 |
| Caprylic/capric triglyceride | 3.0 |
| Beeswax | 4.0 |
| Cetearyl glucoside | 1.5 |
| Sorbitan sesquioleate | 0.9 |
| Vaseline | 3.0 |
| paraffin | 1.5 |
| Preservative, pigment and flavor | adequate |
| Purified water | balance |

[Formulation Example 12] Pack

A pack was prepared according to a commonly employed method with the composition described in Table 9.

[Table 9]

| Ingredient | Content (wt%) |
|---|---|
| Alpine skullcap (*Scutellaria alpina*) extract of Example 1 | 0.2 |
| Glycerin | 4.0 |
| Polyvinyl alcohol | 15.0 |
| Hyaluronic acid extract | 5.0 |
| β-Glucan | 7.0 |
| Allantoin | 0.1 |
| Nonyl phenyl ether | 0.4 |
| Polysorbate 60 | 1.2 |
| Ethanol | 6.0 |
| Preservative, pigment and flavor | adequate |
| Purified water | balance |

While the exemplary embodiments have been shown and described, it will be understood by those skilled in the art that various changes in form and details may be made thereto without departing from the spirit and scope of this disclosure as defined by the appended claims. Accordingly, it is to be understood that the substantial scope of the present disclosure is defined by the appended claims and their equivalents.

[Accession Number]

Depositary authority: Korea Research Institute of Bioscience and Biotechnology.
Accession number: KCTC 12015BP.
Date of accession: Sep. 14, 2011.

## Claims

**1.** A composition for maintaining skin homeostasis, which comprises an alpine skullcap *(Scutellaria alpina)* extract as

an active ingredient.

**2.** A composition for fighting against stress, which comprises an alpine skullcap (*Scutellaria alpina)* extract as an active ingredient.

**3.** A composition for inhibiting the activity of the neuropeptide substance P, which comprises an alpine skullcap (*Scutellaria alpina)* extract as an active ingredient.

**4.** A composition for inhibiting the expression of the NK1R gene or the EDN1 gene, which comprises an alpine skullcap (*Scutellaria alpina)* extract as an active ingredient.

**5.** A composition for enhancing skin immune function, skin moisturizing function or skin barrier function, which comprises an alpine skullcap (*Scutellaria alpina)* extract as an active ingredient.

**6.** A composition for external application to skin, which comprises an alpine skullcap *(Scutellaria alpina)* extract as an active ingredient.

**7.** The composition according to any of claims 1 to 6, wherein the alpine skullcap *(Scutellaria alpina)* is one or more selected from a group consisting of a leaf, a flower, a stem and a root of a plant alpine skullcap (*Scutellaria alpina*).

**8.** The composition according to any of claims 1 to 6, wherein the concentration of the alpine skullcap (*Scutellaria alpina)* extract is 1-50 ppm based on the total volume of the composition.

**9.** The composition according to any of claims 1 to 6, wherein the alpine skullcap *(Scutellaria alpina)* extract is an extract of one or more selected from a group consisting of a water, an organic solvent and a mixture thereof.

**10.** The composition according to claim 9, wherein the organic solvent is one or more selected from a group consisting of a $C_1$-$C_6$ lower alcohol, butylene glycol and propylene glycol.

**11.** The composition according to claim 10, wherein the lower alcohol is ethanol.

**12.** The composition according to any of claims 1 to 5, wherein the composition is a pharmaceutical, food or cosmetic composition.

**13.** The composition according to claim 6, wherein the composition is a pharmaceutical or cosmetic composition.

【FIG. 1】

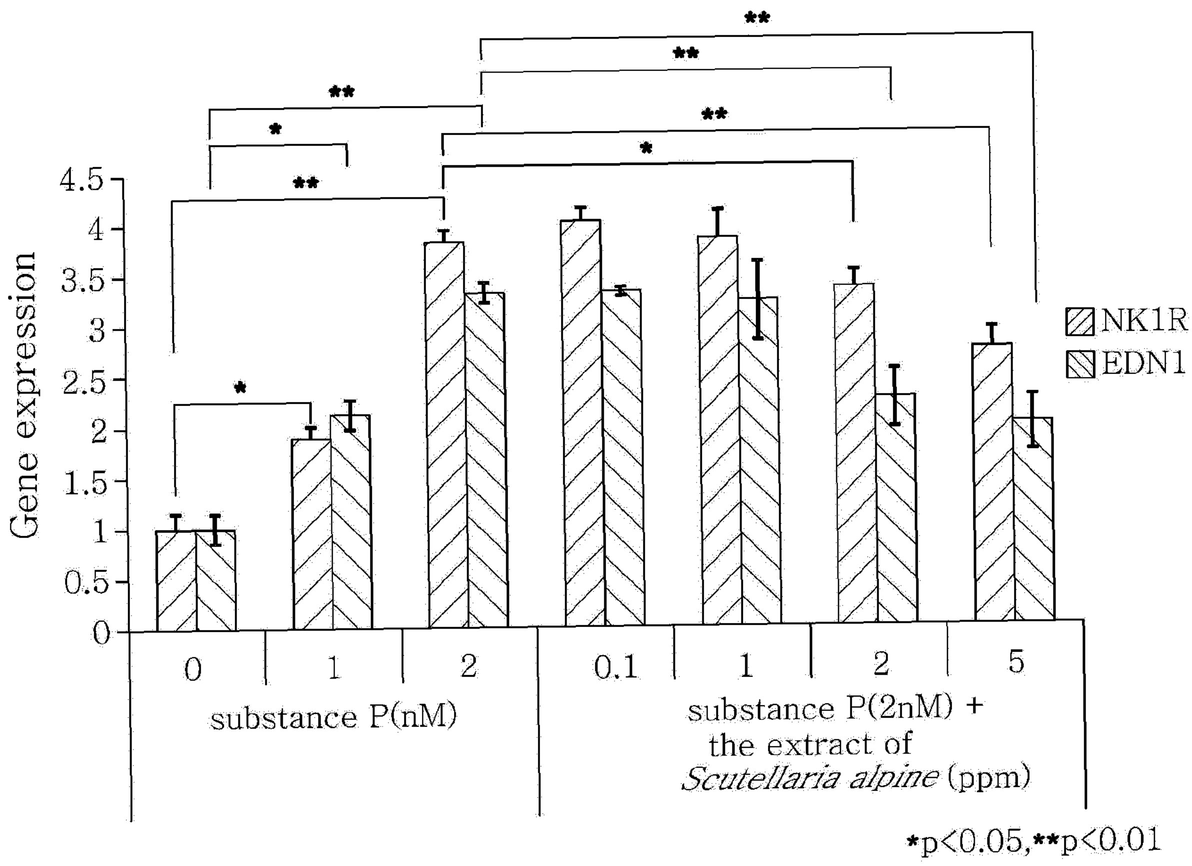

Gene expression
4.5
4
3.5
3
2.5
2
1.5
1
0.5
0
NK1R
EDN1
0
1
2
substance P(nM)
0.1
1
2
5
substance P(2nM) +
the extract of
*Scutellaria alpine* (ppm)
*p<0.05,**p<0.01

【FIG. 2】

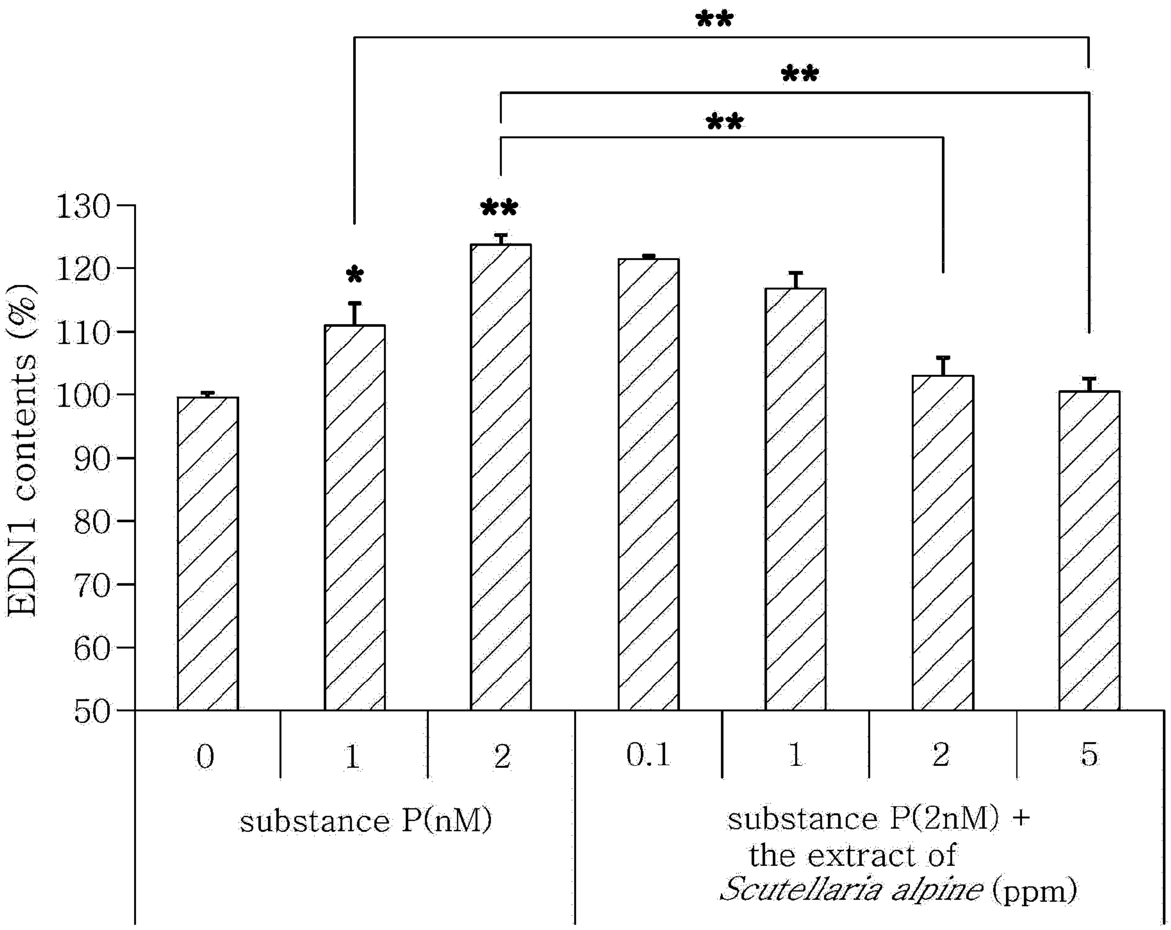


(*p<0.05,**p<0.01)

INTERNATIONAL SEARCH REPORT

International application No.
**PCT/KR2015/004291**

A. CLASSIFICATION OF SUBJECT MATTER

***A61K 36/539(2006.01)i, A61K 8/97(2006.01)i, A23L 1/29(2006.01)i, A61P 17/00(2006.01)i***

According to International Patent Classification (IPC) or to both national classification and IPC

B. FIELDS SEARCHED

Minimum documentation searched (classification system followed by classification symbols)
A61K 36/539; A61K 31/702; A61K 36/48; A61K 8/97; A23L 1/29; A61P 17/00

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: Scutellaria alpina, Substance P, NK1R, END1, skin moisturization, homeostasis, anti-inflammatory

C. DOCUMENTS CONSIDERED TO BE RELEVANT

| Category* | Citation of document, with indication, where appropriate, of the relevant passages | Relevant to claim No. |
|---|---|---|
| X | Alpaflor Scutellaria ao. Datasheet [online]. DSM Nutritional Products Ltd, 2010, pages 1-7 <Product Brochure in URL: http://www.centerchem.com/Products/alpaflor-scuttelaria-ao/> See pages 3-6. | 1-13 |
| Y | SCHAEFER, K., Men's skin care formulating concepts [online], 2010, <URL: http://www.cosmeticsandtoiletries.com/formulating/category/menscare/92809404.html>. See the entire document. | 1-13 |
| Y | US 2008-0020073 A1 (BONTE et al.) 24 January 2008 See abstract; and claims 11-34. | 1-13 |
| A | LEE, H.-J. et al., "Substance P and beta-endorphin mediate electro-acupuncture induced analgesia in mouse cancer pain model", Journal of Experimental & Clinical Cancer Research, 2009, vol. 28, Thesis No. 102, pages 1-9 See abstract. | 1-13 |
| A | XIE, L. et al., "CD10-bearing fibroblasts may inhibit skin inflammation by down-modulating substance P", Archives of Dermatological Research, 2011, vol. 303, pages 49-55 See abstract. | 1-13 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 JULY 2015 (30.07.2015) | **30 JULY 2015 (30.07.2015)** |

| Name and mailing address of the ISA/**KR** | Authorized officer |
|---|---|
| Korean Intellectual Property Office<br>Government Complex-Daejeon, 189 Seonsa-ro, Daejeon 302-701, Republic of Korea<br>Facsimile No. 82-42-472-7140 | Telephone No. |

Form PCT/ISA/210 (second sheet) (July 2009)

**INTERNATIONAL SEARCH REPORT**
Information on patent family members

International application No.
**PCT/KR2015/004291**

| Patent document cited in search report | Publication date | Patent family member | Publication date |
|---|---|---|---|
| US 2008-0020073 A1 | 24/01/2008 | EP 1299081 A1 | 09/04/2003 |
| | | EP 1299081 B1 | 16/01/2008 |
| | | EP 1299081 B9 | 11/03/2009 |
| | | EP 1911494 A2 | 16/04/2008 |
| | | EP 1911494 A3 | 02/07/2008 |
| | | JP 2004-502713 A | 29/01/2004 |
| | | KR 10-0793912 B1 | 15/01/2008 |
| | | KR 10-2003-0062238 A | 23/07/2003 |
| | | US 2003-0157200 A1 | 21/08/2003 |
| | | WO 02-03945 A1 | 17/01/2002 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020140052274
- KR 2012007815 W
- KR 1020110099728

### Non-patent literature cited in the description

- **SELYE, H.** The stress of life. Longnans Green and Co, 1958, 1-50